# EUROPEAN PATENT SPECIFICATION

(11) **EP 2 921 104 B1**
(45) Date of publication and mention of the grant of the patent: **29.03.2017**
(21) Application number: 15156293.1
(22) Date of filing: 24.02.2015
(51) Int. Cl.: A61B 5/0215, A61B 5/00, A61B 5/11

(54) **Blood pressure measuring system**
Blutdruckmesssystem
Dispositif de mesure de pression artérielle

(30) Priority: 18.03.2014 JP 2014054918
(43) Date of publication of application: 23.09.2015
(73) Proprietor: Nihon Kohden Corporation, Shinjuku-ku Tokyo 161-8560 (JP)
(72) Inventor: Kubo, Hiroshi, Tokyo, 161-8560 (JP)
(74) Representative: Hoffmann Eitle

(56) References cited:
- WO-A1-2007/060560
- WO-A2-2011/061732
- JP-A- 2006 102 190
- US-A1- 2005 197 585

## Description

### BACKGROUND

The invention relates to a blood pressure measuring system which invasively measures the blood pressure of the subject.

There is an invasive blood pressure measuring method in which a catheter or the like is inserted into the blood vessel of the subject, and the blood pressure changing every moment is continuously measured (for example, see JP-A-4-269938). The pressure which is generated in a blood vessel is converted into an electrical signal by a transducer which is connected to the catheter via a monitoring line. A blood pressure monitor displays the blood pressure value and waveform corresponding to the electrical signal, to a medical person or the like.

In an invasive blood pressure measurement, it is necessary to perform a process called the zero point calibration for setting a reference value of the measurement. The zero point calibration is performed in the following manner. The transducer is placed in a reference point. When a pressure acting on the transducer is made zero (when the catheter is opened to the atmosphere), the electrical signal indicative of the blood pressure value is set to have a measurement reference value.

The zero point calibration is performed each time the subject is changed. Even during a measurement on the same subject, however, the measurement reference value is caused to vary (drift) by various causes. The largest variation cause is that the position relationship (relative altitude) of the blood pressure measurement position and the transducer is changed by a postural change of the subject or the like. When the measurement reference value varies, the blood pressure measurement is not correctly conducted, and therefore the zero point calibration must be again performed. In the zero point calibration, a three-way stopcock to which the transducer is connected must be opened to the atmosphere. A large burden is imposed to both the subject and the medical person by performing the zero point calibration each time the posture of the patient is changed.

WO 2007/060560 A1 relates to a method for adjusting a blood pressure measurement to compensate for a pressure offset due to obtaining the blood pressure measurement at an elevation other than heart level is illustrated. The method includes measuring a blood pressure at a location above or below a heart level and adjusting the measured blood pressure in accordance with a pressure difference between the location at which the blood pressure is obtained and the heart level by concurrently measuring a hydrostatic offset and blood pressure and combining (e.g., adding, subtracting, etc.) the offset with the blood pressure to generate an offset-adjusted blood pressure.

WO 2011/061732 A2 relates to a method for automatically deriving a correct blood pressure measurement of a patient, by first ensuring that at least two conditions are fulfilled: first, that the patient is in a state of rest and second, that blood pressure is measured at heart level height. The first condition is achieved when the measured pulse rate is stable, and the second condition is achieved by deriving the heart height level of the patient and then disposing the blood pressure sensor at heart height level. A heart-height derivation device is configured to derive a front view image of the patient, and to run a computer program for deriving the heart height level of the patient from the front view image.

### SUMMARY

The invention may provide a blood pressure measuring system capable of reducing the burden which is imposed to the subject and a medical person in an invasive blood pressure measurement.

The blood pressure measuring system has the features defined in claim 1. The dependent claims describe advantageous embodiments.

The blood pressure measuring system may comprise: a blood pressure monitor which is configured to invasively measure a blood pressure value of a subject; a calibrating section which
is configured to calibrate a measurement reference value of the blood pressure value; an imaging section which is configured to acquire an image of the subject; a heart position identifying section which is configured to identify a height position of a heart of the subject based on the image; and a determining section which, based on the identified height position of the heart, is configured to determine whether a calibration of the measurement reference value is necessary or not.

When the determining section determines that the calibration is necessary, the calibrating section may automatically calibrate the measurement reference value.

The blood pressure measuring system may further comprise: a notifying section which, when the determining section determines that the calibration is necessary, is configured to output an alarm.

The blood pressure measuring system may further comprise: a reference member which is adapted to be attached to the subject, and which is to be imaged by the imaging section.

The imaging section may include a plurality of cameras which take images of the subject in a plurality of directions.

### BRIEF DESCRIPTION OF THE DRAWINGS

Fig. 1 is a view schematically showing a blood pressure measuring system of an embodiment of the invention.
Figs. 2A and 2B are views illustrating a process of calibrating a blood pressure measurement reference value in the blood pressure measuring system.

### DETAILED DESCRIPTION OF EXEMPLARY EMBODIMENTS

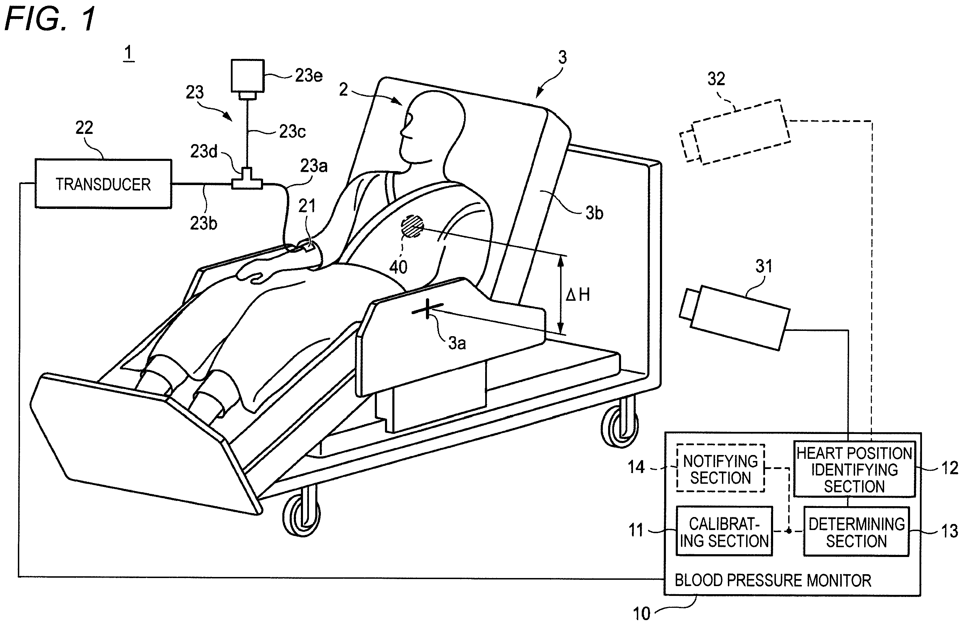

An embodiment of the invention will be described in detail with reference to the accompanying drawing. Fig. 1 is a view schematically showing a blood pressure measuring system 1 of the embodiment of the invention. In the figure, the scale is adequately changed in order to draw components in a recognizable size.

The blood pressure measuring system 1 includes a blood pressure monitor 10. The blood pressure monitor 10 invasively measures the blood pressure value of the subject 2.

The illustrated embodiment shows the case where the arterial blood pressure is measured. A catheter (arterial needle) 21 is inserted into the radial artery of the subject 2. In the case where the venous blood pressure is measured, a Swan-Ganz catheter may be used. A transducer 22 is fixed at the level of the heart (the level corresponding to a half of the chest depth) of the subject 2. The catheter 21 and the transducer 22 are connected to each other through a monitoring line 23.

The monitoring line 23 includes a first tube 23a, a second tube 23b, a third tube 23c, a three-way stopcock 23d, and an infusion bottle 23e. The first tube 23a connects the catheter 21 to the three-way stopcock 23d. The second tube 23b connects the transducer 22 to the three-way stopcock 23d. The third tube 23c connects the infusion bottle 23e to the three-way stopcock 23d. The infusion bottle 23e stores a physiological saline solution containing heparin. When the three-way stopcock 23d is opened in all the directions, the first tube 23a, the second tube 23b, and the third tube 23c are filled with the physiological saline solution containing heparin.

The transducer 22 outputs an electrical signal corresponding to the pressure in the blood vessel of the subject 2, the pressure being transmitted through the physiological saline solution containing heparin. The blood pressure monitor 10 displays the blood pressure value and waveform corresponding to the electrical signal, to the user such as a medical person.

The blood pressure measuring system 1 includes a calibrating section 11. The calibrating section 11 is configured so as to calibrate the measurement reference value of the blood pressure value measured by the blood pressure monitor 10. When the measurement of the blood pressure is to be started, an initial zero point calibration of the blood pressure monitor 10 is performed. Specifically, the catheter 21 is set to an atmosphere open state to make the pressure acting on the transducer 22 zero. In this state, the calibrating section 11 performs a calibration so that the electrical signal supplied to the blood pressure monitor 10 has a reference value.

The blood pressure measuring system 1 includes a camera 31 (an example of the imaging section). The camera 31 is configured so as to acquire an image of the subject 2.

The blood pressure measuring system 1 further includes a heart position identifying section 12. The heart position identifying section 12 is configured so as to identify the height position of the heart of the subject 2 based on an image acquired by the camera 31.

Specifically, the difference ΔH between a predetermined reference height and the height of the heart of the subject 2 is identified. Examples of the predetermined reference height are the height at which the transducer 22 is placed, and that of an index 3a disposed on a bed 3 on which the subject 2 is lying. The index 3a may be set so as to indicate the height of the transducer 22.

The heart position identifying section 12 identifies the position of the heart of the subject 2 in the image by using the image recognition technique. For example, the section may be configured so that the section detects the head and shoulder of the subject 2, and identifies the position of the heart from the positions of the head and the shoulder. On the other hand, the heart position identifying section 12 identifies the positions of the transducer 22 and the index 3a by using the image recognition technique. The heart position identifying section 12 previously stores information related to the positional relationships (the distance, the imaging angle, and the like) between the subject 2 and the camera 31. Based on the reference height and height of the heart of the subject 2 which are identified as described above, the heart position identifying section 12 identifies the difference ΔH between the heights.

The blood pressure measuring system 1 includes a determining section 13. The determining section 13 is configured so as to, based on the difference ΔH acquired by the heart position identifying section 12, determine whether a calibration of the measurement reference value of the blood pressure monitor 10 is necessary or not. In the case where, at the start of the measurement, the value of the difference ΔH is varied at a degree which is larger than a predetermined value, for example, it is determined that a calibration is necessary. The predetermined value is set to a value of a degree by which the execution of a correct measurement of the blood pressure value is impeded. In other words, even in the case where a postural change of the subject 2 occurs, when the change of the difference value is smaller than the predetermined value, the determining section 13 does not determine that a calibration is necessary.

The subject 2 shown in Fig. 1 is lying on the reclining bed 3. The angle of the backrest 3b of the bed 3 is variable so that the upper body of the patient 2 can be raised to a desired angle. Also in such a case, the variation of the difference ΔH may exceed the predetermined value. Therefore, the determining section 13 determines that a calibration of the measurement reference value of the blood pressure monitor 10 is necessary.

According to the configuration, based on the image which is acquired by the camera 31, it is possible to grasp only the variation of the measurement reference value caused by a postural change of the subject 2. Therefore, an appropriate response is possible. When the degree of the postural change is within the range where the blood pressure measurement is not impeded, it is possible to determine that the calibration is not necessary. Consequently, the opportunity to perform the zero point calibration involving an operation of opening the three-way stopcock 23d to the atmosphere can be reduced, and the burden which is imposed to the subject and the medical person in the invasive blood pressure measurement can be reduced.

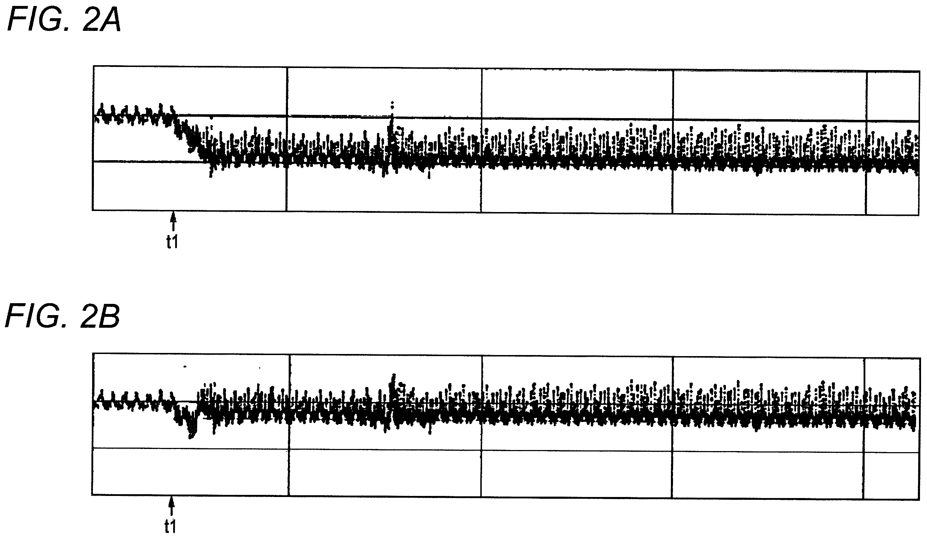

The calibrating section 11 is configured so as to execute a calibration of the measurement reference value through a dial or switch which can be manually operated by the user. Fig. 2A shows the waveform of the central venous pressure (CVP) acquired from the subject 2. The abscissa represents the time, and the ordinate represents the signal intensity. The reduction of the measured value of the blood pressure at time t1 is caused by a postural change of the subject 2. Fig. 2B shows the waveform of the central venous pressure (CVP) in which a calibration of the measurement reference value is executed by the calibrating section 11. Although a temporary reduction of the measured value of the blood pressure caused by a postural change of the subject 2 is observed at time t1, it is seen that the measured value is returned by the calibration to the level obtained before time t1.

The target of the calibration is not limited to the central venous pressure (CVP). Various waveforms representing a blood pressure which is obtained by an invasive blood pressure measurement may be used as the target of the calibration.

As indicated by the broken line in Fig. 1, alternatively, the calibrating section 11 and the determining section 13 may be communicably connected to each other. In the alternative, the determining section 13 supplies a signal indicative of the necessity/unnecessity of a calibration of the measurement reference value, to the calibrating section 11. The calibrating section 11 is configured so as to, when the determining section 13 determines that a calibration is necessary, automatically set the measurement reference value. For example, relationships between the value of the difference ΔH which is identified by the heart position identifying section 12, and the correction amount of the measurement reference value are previously stored in the calibrating section 11 in the form of a table or a function. When the determining section 13 determines that a calibration is necessary, the calibrating section 11 refers the value of the difference ΔH identified by the heart position identifying section 12, and determines the correction amount of the measurement reference value based on the table or the function.

According to the configuration, the calibration of the measurement reference value is automated, and the necessity to perform the zero point calibration involving an operation of opening the three-way stopcock 23d to the atmosphere can be further suppressed. Therefore, the burden which is imposed to the subject and the medical person in the invasive blood pressure measurement can be further reduced.

As indicated by the broken lines in Fig. 1, the blood pressure measuring system 1 may include a notifying section 14. In this case, the notifying section 14 is configured so as to output an alarm when the determining section 13 determines that a calibration is necessary.

According to the configuration, in the case where the calibration of the measurement reference value is to be manually performed, the necessity can be notified more surely to the medical person. In the case where the calibration of the measurement reference value is to be automatically performed by the calibrating section 11, the medical person can easily grasp the situation where the calibration process is performed (i.e., the system is not in the usual measuring state). Therefore, the burden which is imposed to the medical person in an invasive blood pressure measurement can be further reduced.

As indicated by the hatched lines in Fig. 1, a reference member 40 which is to be imaged by the camera 31 may be attached to the subject 2. The shape and color of the reference member 40 are determined so as to assist in the image recognition performed by the heart position identifying section 12. The attachment position of the reference member 40 may be adequately determined. However, it is preferable to determine a position corresponding to the heart of the subject 2 as the attachment position.

According to the configuration, the reference member 40 is moved following a postural change of the subject 2, and therefore the position of the heart of the subject 2 can be identified more surely through image recognition. This improves the accuracy of the determination by the determining section 13 whether the calibration of the measurement reference value is necessary or not. Therefore, the burden which is imposed to the medical person in an invasive blood pressure measurement can be further reduced.

As indicated by the broken lines in Fig. 1, the blood pressure measuring system 1 may further include a camera 32. The camera 32 is configured so as to take an image of the subject 2 in a different direction from that of the camera 31. Namely, the subject 2 is imaged in a plurality of directions by a plurality of cameras (an example of the imaging portion). Image data acquired by the camera 32 are supplied to the heart position identifying section 12, and then processed in the same manner as the image data acquired by the camera 31.

In the case of the imaging using a single camera, depending on the posture of the subject 2, there is a possibility that the image recognition process may be hardly performed, or the reference member 40 may be hidden. When the subject 2 is imaged in a plurality of directions, the position of the heart of the subject 2 can be identified more surely. This improves the accuracy of the determination by the determining section 13 whether the calibration of the measurement reference value is necessary or not. Therefore, the burden which is imposed to the medical person in an invasive blood pressure measurement can be further reduced.

The embodiment has been described in order to facilitate understanding of the invention, and is not intended to limit the invention. It is a matter of course that the invention may be changed or improved without departing the spirit thereof, and includes equivalent of the embodiment.

In the example shown in Fig. 1, the calibrating section 11, the heart position identifying section 12, the determining section 13, and the notifying section 14 are disposed in the blood pressure monitor 10. However, at least one of these sections may be disposed in an apparatus other than the blood pressure monitor 10.

It is not always required that the blood pressure monitor 10 is an independent apparatus. The blood pressure monitor may be provided as one function realized in a biological information monitoring apparatus which acquires and displays biological information such as an electrocardiogram.

According to the presently subject matter, there is provided a blood pressure measuring system comprising: a blood pressure monitor which is configured to invasively measure a blood pressure value of a subject; a calibrating section which is configured to calibrate a measurement reference value of the blood pressure value; an imaging section which is configured to acquire an image of the subject; a heart position identifying section which is configured to identify a height position of a heart of the subject based on the image; and a determining section which, based on the identified height position of the heart, is configured to determine whether a calibration of the measurement reference value is necessary or not.

According to the above configuration, based on the image which is acquired by the imaging section, it is possible to grasp only the variation of the measurement reference value caused by a postural change of the subject. Therefore, an appropriate response is possible. When the degree of the postural change is within the range where the blood pressure measurement is not impeded, it is possible to determine that the calibration is not necessary. Consequently, the opportunity to perform the zero point calibration involving an operation of opening a three-way stopcock to the atmosphere can be reduced, and the burden which is imposed to the subject and a medical person in an invasive blood pressure measurement can be reduced.

When the determining section determines that the calibration is necessary, the calibrating section may automatically calibrate the measurement reference value.

According to the above configuration, the calibration of the measurement reference value is automated, and the necessity to perform the zero point calibration involving an operation of opening the three-way stopcock to the atmosphere can be further suppressed. Therefore, the burden which is imposed to the subject and a medical person in an invasive blood pressure measurement can be further reduced.

The blood pressure measuring system may further include a notifying section which, when the determining section determines that the calibration is necessary, is configured to output an alarm.

According to the above configuration, in the case where the calibration of the measurement reference value is to be manually performed, the necessity can be notified more surely to a medical person. In the case where the calibration of the measurement reference value is to be automatically performed by the calibrating section, a medical person can easily grasp the situation where the calibration process is performed (i.e. , the system is not in the usual measuring state). Therefore, the burden which is imposed to the medical person in an invasive blood pressure measurement can be further reduced.

The blood pressure measuring system may further include a reference member which is adapted to be attached to the subject, and which is to be imaged by the imaging section.

According to the above configuration, the reference member is moved following a postural change of the subject, and therefore the position of the heart of the subject can be identified more surely through image recognition. This improves the accuracy of the determination by the determining section whether the calibration of the measurement reference value is necessary or not. Therefore, the burden which is imposed to the medical person in an invasive blood pressure measurement can be further reduced.

The imaging section may include a plurality of cameras which take images of the subject in a plurality of directions.

In the case of the imaging using a single camera, depending on the posture of the subject, there is a possibility that the image recognition process may be hardly performed, or the reference member may be hidden. When the subject is imaged in a plurality of directions, the position of the heart of the subject can be identified more surely. This improves the accuracy of the determination by the determining section whether the calibration of the measurement reference value is necessary or not. Therefore, the burden which is imposed to the medical person in an invasive blood pressure measurement can be further reduced.

## Claims

1. A blood pressure measuring system comprising:
a blood pressure monitor (10) which is configured to invasively measure a blood pressure value of a subject;
a calibrating section (11) which is configured to calibrate a measurement reference value of the blood pressure value;
**characterized in that** the system further comprises:
an imaging section (31) which is configured to acquire an image of the subject;
a heart position identifying section (12) which is configured to identify a height position of a heart of the subject with respect to a predetermined reference height based on the image; and
a determining section (13) which, based on the identified height position of the heart, is configured to determine whether a calibration of the measurement reference value is necessary or not.

2. The blood pressure measuring system according to claim 1, wherein, when the determining section (13) determines that the calibration is necessary, the calibrating section automatically calibrates the measurement reference value.

3. The blood pressure measuring system according to claim 1 or 2, further comprising: a notifying section (14) which, when the determining section determines that the calibration is necessary, is configured to output an alarm.

4. The blood pressure measuring system according to any one of claims 1 to 3, further comprising: a reference member (40) which is adapted to be attached to the subject, and which is to be imaged by the imaging section.

5. The blood pressure measuring system according to any one of claims 1 to 4, wherein the imaging section includes a plurality of cameras (31, 32) which take images of the subject in a plurality of directions.

## Patentansprüche

1. Blutdruckmesssystem, umfassend:
eine Blutdruck-Überwachungsvorrichtung (10), die eingerichtet ist zum invasiven Messen eines Blutdruckwertes eines Subjekts;
einen Kalibrierungsabschnitt (11), der eingerichtet ist zum Kalibrieren eines Meßreferenzwertes des Blutdruckwertes;
**dadurch gekennzeichnet, dass** das System weiterhin umfasst:
einen Abbildungsabschnitt (31), der eingerichtet ist zum Erfassen eines Bildes des Subjekts;
einen Herzposition-Identifikationsabschnitt (12), der eingerichtet ist zum Identifizieren einer Höhenposition eines Herzens des Subjekts mit Bezug auf eine vorbestimmte Referenzhöhe basierend auf dem Bild; und
einen Bestimmungsabschnitt (13), der, basierend auf der identifizierten Höhenposition des Herzens, eingerichtet ist zum Bestimmen, ob eine Kalibration des Meßreferenzwertes nötig ist oder nicht.

2. Blutdruckmesssystem nach Anspruch 1, wobei, wenn der Bestimmungsabschnitt (13) bestimmt, dass die Kalibration nötig ist, der Kalibrierungsabschnitt automatisch den Meßreferenzwert kalibriert.

3. Blutdruckmesssystem nach Anspruch 1 oder 2, das weiterhin umfasst: einen Meldeabschnitt (14), der, wenn der Bestimmungsabschnitt bestimmt, dass die Kalibration nötig ist, eingerichtet ist zum Ausgeben eines Alarms.

4. Blutdruckmesssystem nach einem der Ansprüche 1 bis 3, das weiterhin umfasst: ein Referenzbauteil (40), das eingerichtet ist, an dem Subjekt angebracht zu sein, und das von dem Abbildungsabschnitt abzubilden ist.

5. Blutdruckmesssystem nach einem der Ansprüche 1 bis 4, wobei der Abbildungsabschnitt eine Vielzahl von Kameras (31, 32) enthält, die Bilder des Subjekts in eine Vielzahl von Richtungen nimmt.

## Revendications

1. Système de mesure de pression artérielle comprenant :
un moniteur de pression artérielle (10) qui est configuré pour mesurer de manière invasive une valeur de pression artérielle d'un sujet;
une section de calibrage (11) qui est configurée pour calibrer une valeur de référence de mesure de la valeur de pression artérielle;
**caractérisé en ce que** le système comprend en outre :
une section d'imagerie (31) qui est configurée pour acquérir une image du sujet;
une section d'identification de position de coeur (12) qui est configurée pour identifier une position en hauteur du coeur du sujet par rapport à une hauteur de référence prédéterminée sur la base de l'image ; et
une section de détermination (13)qui, sur la base de la position en hauteur identifiée du coeur, est configurée pour déterminer si un calibrage de la valeur de référence de mesure est nécessaire ou pas.

2. Système de mesure de pression artérielle selon la revendication 1, dans lequel, lorsque la section de détermination (13) détermine que le calibrage est nécessaire, la section de calibrage calibre automatiquement la valeur de référence de mesure.

3. Système de mesure de pression artérielle selon la revendication 1 ou 2, comprenant en outre : une section de notification (14) qui, lorsque la section de détermination détermine que le calibrage est nécessaire, est configurée pour émettre une alarme.

4. Système de mesure de pression artérielle selon l'une quelconque des revendications 1 à 3, comprenant en outre : un membre de référence (40) qui est adapté pour être attaché au sujet, et qui doit être reproduit par imagerie par la section d'imagerie.

5. Système de mesure de pression artérielle selon l'une quelconque des revendications 1 à 4, dans lequel la section d'imagerie inclut une pluralité de caméras (31, 32) qui prennent des images du sujet dans une pluralité de directions.
